# EUROPEAN PATENT SPECIFICATION

(11) **EP 1 512 387 B1**
(45) Date of publication and mention of the grant of the patent: **02.02.2011**
(21) Application number: 03752925.2
(22) Date of filing: 20.05.2003
(51) Int. Cl.: A61F 13/496

(54) **PANTY TYPE DIAPER**
WINDEL VOM HÖSCHENTYP
COUCHE-CULOTTE DE TYPE CULOTTE

(30) Priority: 22.05.2002 JP 2002147758
(43) Date of publication of application: 09.03.2005
(73) Proprietor: UNI-CHARM CORPORATION, Kawanoe-shi Ehime 799-0111 (JP)
(72) Inventor: SUZUKI, Seiji, Technical Center, Uni-Charm Co., Mitoyo-gun, Kagawa 769-1602 (JP); SAKAGUCHI, Satoru, Technical Center, Uni-Charm Co., Mitoyo-gun, Kagawa 769-1602 (JP)
(74) Representative: Fitchett, Stuart Paul
(86) International application number: PCT/JP2003/006291
(87) International publication number: WO 2003/096948

(56) References cited:
- EP-A- 0 323 040
- EP-A- 0 878 180
- JP-A- 3 176 052
- JP-A- 2002 242 001
- JP-U- 54 125 711
- JP-U- 56 126 403

## Description

### FIELD OF THE INVENTION

The invention relates to a brief-type diaper, and more particularly, to a diaper which is molded into the form of a brief by folding an absorbent for absorbing body fluids in a multilayer at the center of a pre-form blank for a brief-type diaper and by cementing together side edge portions of the pre-form blank for a brief-type diaper.

### RELATED ART

A diaper, such as that shown in, e.g., Fig. 12, is available as a diaper of this type (see JP 06-296638A). The drawing shows a sheet into which the diaper is developed. A bow-shaped notch "b" is formed at either side edge of a sheet "a." An absorbent "e" is provided so as to spread across a front abdominal region "c" and a rear abdominal region "d." The sheet "a" is vertically folded in two, and side edge portions "f" are cemented together, thereby forming a waist opening and leg openings.

However, in the case of this diaper, side edge portions "f" of the respective front and rear abdominal regions "c" and "d" are cemented together, and hence cemented lines possessing rigidity are present so as to extend from the waist opening to the leg openings. For this reason, a wearer has an unusual feeling around his/her girth and legs.

When the diaper is worn, the diaper is often pulled up by pinching lateral portion of the waist opening with fingers, although rigidity inherent of cemented section will result in difficulty for the diaper to be pulled up by pinching the waist opening.

In the case of the diaper of this type, the absorbent is formed flat, and hence excretions, such as fecal material, are likely to adhere to the wearer's skin. Therefore, the applicant have invented a disposable diaper (as described in JP2000-225143A) comprising a top sheet, a back sheet, and an absorbent sandwiched therebetween. The diaper further comprises a V-shaped recessed notch section extending from a rear girth region to a crotch region. Side edges of the V-shaped recessed notch are cemented together, thereby forming a pocket (recess) for holding excretions within the diaper.

However, the invention involves a necessity for a process of cutting a laminated panel into the shape of the letter V from a rear girth region to the crotch region, to thereby form a recessed notch, and a process of fixedly cementing together the side edge portions of the recessed notch. Thus, a diaper has hitherto been formed not simultaneously with formation of a pocket.

### DISCLOSURE OF THE INVENTION

The invention has been conceived against the foregoing backdrop and provides a brief-type diaper which eliminates unusual feeling, which would otherwise arise when legs are moved, improves ease of wearing, and forms a pocket for holding excretions simultaneous with formation of a diaper.

In order to solve the problem, the present inventors have found that a brief-type diaper having no cemented sections in right and left abdominal regions within a girth section is obtained by horizontally folding a pre-form blank for the diaper along the axis of symmetry and cementing side edge portions of the pre-form blank for a diaper, wherein the pre-form blank for a brief-type diaper has an outer sheet constituting an exterior, an inner sheet which comes into contact with a wearer, an absorbent for absorbing body fluids, an elastic member for causing portions of the outer and inner sheets to contract and extend, and a pair of leg openings through which the wearer's legs penetrate, and forming a pocket for holding excretions simultaneously with formation of the brief-type diaper. On the basis of this finding, the present inventors have come to complete the present invention.

Further, the brief-type diaper of the invention is provided on the basis of the novel idea that a pair of leg openings are formed in advance in a pre-form blank for a brief-type diaper and the side edge portions are cemented together by folding along an intermediate position between the pair of leg openings.

More specifically, the present invention provides the following:
(1) A brief-type diaper having an outer sheet constituting an exterior, an inner sheet which comes into contact with a wearer, an absorbent for absorbing body fluids, an elastic member which Is provided on either the inner sheet or the outer sheet or on both sheets and which causes portions of the outer and inner sheets to contract and extend, and a pair of leg openings through which the wearer's legs penetrate, the diaper comprising a cemented section which extendser's from a waist opening to a crotch section while avoiding the leg openings in a front or rear abdominal region of the brief-type diaper.
   According to the invention (1), the cemented section is provided so as to extend from the waist opening to the crotch section in the front or rear abdominal region while avoiding the leg openings. Therefore, no cemented sections are provided on right and left abdominal regions of the girth section (specifically, the both side portions of girth section) so as to extend from the waist opening to the crotch section. Therefore, when a wearer wears the diaper, the diaper is readily pulled up while pinching the lateral portion of the waist opening with the fingers. Thus, the diaper becomes easier to wear. Therefore, the wearer does not have any unusual feeling upon wearing them, which would otherwise arise in a girth or around the legs.
   In the invention, the term "front abdominal region" means an abdominal-side portion of a brief-type diaper when the diaper is viewed from the front. The term "rear abdominal region" means a back side opposite to the front abdominal region. Also the term "right and left abdominal regions" means lateral portion of side body surface when the brief-type diaper is viewed from front.
(2) The brief-type diaper according to (1), wherein the cemented section is a continuous fiber which extends across a substantial center section of the front or rear abdominal region in a direction in which the brief-type diaper is to be worn.
   According to the invention (2), the cemented section is a continuous fiber which extends across a substantial-center section of the front or rear abdominal region in a direction in which the brief-type diaper is to be worn. The cemented section is provided straight in the front or rear abdominal region from the waist opening to the crotch and no cemented section are provided on the girth region, thus the wearer does not have any unusual feeling upon wearing them. Also, no cemented sections are provided on right and left abdominal regions of the girth section (more specifically, both side area of girth section) so as to extend from the waist opening to the leg openings, thus allowing the diaper to be readily pulled up while pinching the right and left side portion of the waist opening with the fingers as he/she pulls up the diaper.
   Here, the term "direction in which the brief-type diaper is to be worn" means to describe the vertical direction as he/she pulls up and down the diaper, while wearer stands upon wearing the diaper. The term "fiber" described as not being particularly limited to any thickness or width, so long as to form a continuous fine string-like or thin elongated band-like condition.
(3) A pre-form blank for a brief-type diaper which is formed from a substantially-W-shaped single cloth made by cutting one side of a rectangle in the form of twin peaks and which has a pre-girth section to act as a girth section, pre-leg opening sections to act as leg opening sections, and a pre-crotch section to act as a crotch section, further comprising the pre-girth section being present in the form of a substantially-rectangular portion, wherein an elastic member is provided in the pre-girth section in a stretched manner. Also the pre-leg openings being present in the form of a pair holes and at positions which are symmetrical about the axis of symmetry of the pre-form blank for a brief-type diaper. The pre-crotch section being present in the form of twin peaks extending from a lower edge of the substantially-rectangular pre-girth section.
   According to the pre-form blank for a brief-type diaper of the invention (3), wherein the pre-form blank for a brief-type diaper is formed from a substantially-W-shaped single cloth made by cutting one side of a rectangle in the form of twin peaks and has a pre-girth section to act as a girth section, pre-leg opening sections to act as leg opening sections, and a pre-crotch section to act as a crotch section. Moreover, the pre-form blank further comprises the pre-girth section being present in the form of a substantially-rectangular portion, wherein an elastic member is provided in the pre-girth section in a stretched manner; the pre-leg openings being present in the form of a pair holes and at positions which are symmetrical about the axis of symmetry of the pre-form blank for a brief-type diaper; and the pre-crotch section being present in the form of twin peaks extending from a lower edge of the substantially-rectangular pre-girth section.
   The absorbent is arranged on the pre-form blank for a brief-type diaper so as to extend from an intermediate position between the pre-leg opening sections along a lower edge of the pre-crotch section. The pre-form blank for a brief-type diaper is horizontally folded along the axis of symmetry of the pre-form blank. The side edge portions of the superimposed pre-girth section and lower edge portions of the pre-crotch section assuming the shape of twin peaks are cemented together, thereby forming a waist opening. Thus, a brief-type diaper is formed. Here, the pre-form blank is folded and cemented along an intermediate section between the pair of pre-leg openings (More specifically, the axis of symmetry of the pre-form blank for a diaper). As a result, a cemented section is formed in the rear or front abdominal region.
   In the invention, the pre-form blank is a member to be used for forming a brief-type diaper and signifies a cloth-like member into which the diaper has been developed by exfoliating the cemented section. Here, the term "hole of pre-leg opening section" means a hole which forms a leg opening when the pre-form blank is formed into a diaper. The geometry of the pre-leg opening section is not limited to a substantial crescent shape but may be circular or may take the form of a simple cut line (slit).
(4) A pre-form blank for a brief-type diaper which is formed from a single cloth, the cloth assuming a substantially-semicircular shape and having a notch section at substantially the center of a chord of the semicircular pattern, and which has a pre-girth section to act as a girth section, pre-leg opening sections to act as leg opening sections, and a pre-crotch section to act as a crotch section, and further comprising the pre-girth section and the pre-crotch section being located as a chord of the semicircular pattern, in which an elastic member is provided in the pre-girth section in an elongated state; and the pre-leg openings being present in the form of holes and at positions which are symmetrical about the axis of symmetry of the pre-form blank for a brief-type diaper.
   The pre-form blank for a brief-type diaper according to (4), formed from a single cloth, the cloth assuming a substantially-semicircular shape and having a notch section at substantially the center of a chord of the semicircular pattern, and has a pre-girth section to act as a girth section, pre-leg opening sections to act as leg opening sections, and a pre-crotch section to act as a crotch section and further includes the pre-girth section and the pre-crotch section being located as a chord of the semicircular pattern, in which an elastic member is provided in the pre-girth section in an elongated state; and the pre-leg openings being present in the form of a pair holes and at positions which are symmetrical about the axis of symmetry of the pre-form blank for brief-type diaper.
   Therefore, the absorbent is arranged on the pre-form blank for a brief-type diaper so as to extend from an intermediate position between the pre-leg opening sections along the chord of the semicircular pattern. The pre-form blank for a brief-type diaper is horizontally folded along the axis of symmetry of the pre-form blank. The lower edge portions of the superimposed semicircular chord are cemented together, thereby forming a waist opening. Thus, a brief-type diaper is formed. Here, the pair of pre-leg openings (the axis of symmetry of the pre-form blank for a diaper) is folded and cemented along the intermediate section between the pre-leg openings. As a result, a cemented section is formed in the rear or front abdominal region.
(5) A pre-form blank for a brief-type diaper which is formed from a single cloth, the cloth assuming a substantially-rectangular shape and having a substantially-trapezoidal protrusion projecting from a center of a lower edge of the rectangular edge, and which has a pre-girth section to act as a girth section, pre-leg opening sections to act as leg opening sections, and a pre-crotch section to act as a crotch section, and further comprising the pre-girth section being present in the form of a substantial-rectangular portion, wherein an elastic member is provided in the pre-girth section in a stretched manner and the pre-leg openings being present in the form of a peak-shaped notch in the pre-girth section and the pre-crotch section and straddling the substantially-trapezoidal protrusion; and the pre-crotch section being present in the form of a substantially-trapezoidal protrusion extending from a center of a lower edge of the substantially-rectangular pre-girth section.
   The pre-form blank for a brief-type diaper of the invention (5) is a single cloth, assuming a substantially-rectangular shape and having a substantially-trapezoidal protrusion projecting from a center of a lower edge of the substantially-rectangular edge, and has a pre-girth section to act as a girth section, pre-leg opening sections to act as leg opening sections, and a pre-crotch section to act as a crotch section and further includes the pre-girth section being present in the form of a substantially-rectangular portion, wherein an elastic member is provided in the pre-girth section in a stretched manner; the pre-leg openings being present in the form of a peak-shaped notch in the pre-girth section and the pre-crotch section and straddling the substantially-trapezoidal protrusion; and the pre-crotch section being present in the form of a substantially-trapezoidal protrusion extending from a center of a lower edge of the rectangular pre-girth section.
   Therefore, the upper half of the absorbent is provided at an intermediate position between the pre-leg opening sections of pre-crotch section (more specifically, an intermediate position between the notches) in the pre-form blank for a brief-type diaper. The pre-form blank for a brief-type diaper is horizontally folded along the axis of symmetry of the pre-form blank. The superimposed side edge portions of the pre-girth section, lower edge portions of the pre-crotch section, and lower edge portions of the pre-girth section are cemented together. A waist opening and pre-leg openings are formed, thus, a brief-type diaper is formed. Further, the pre-form blank is folded and cemented along an intermediate section between the pair of pre-leg openings (the axis of symmetry of the pre-form blank for a diaper). As a result, a cemented section is formed in the rear or front abdominal region.
(6) The brief-type diaper according to (1) or (2), wherein the absorbent is split in two from a center thereof to one longitudinal end thereof in a widthwise direction; a non-split section of the absorbent is located at an intermediate section between the pair of leg openings in the front abdominal region; and the split portions of the absorbent are provided at a substantial center of the rear abdominal region and on both sides of and along the cemented section.
   According to the invention (6), the absorbent is split in to two from a center thereof to one longitudinal end thereof in a widthwise direction. A non split section of the absorbent is located at a substantially-center of the front abdominal region and at the same time a split section of the absorbent is located at a substantially-center of the rear abdominal region along both sides of the cemented section (more specifically, on the right and left sides of the cemented section).
   Therefore, the cemented section is provided at the center of the rear abdominal region. The split portions of the absorbent are placed on the right and left sides of the cemented section. By means of cementing operation required for forming a brief-type diaper from the pre-form blank, the absorbent is pulled, whereby a bulge is formed from the absorbent in the crotch section in the rear abdominal region. By means of the bulge, excretions, such as fecal matter, are accommodated and held such that the excretions do not touch the wearer's body.
(7) The brief-type diaper according to (1) or (2), wherein the absorbent is split in two from a center thereof to one longitudinal end thereof in a widthwise direction; a non-split section of the absorbent is located at a substantially center of the rear abdominal region, and the split portions of the absorbent are provided at an intermediate section between the leg openings in the front abdominal region and on both sides of and along the cemented section.
   According to the invention (7), the absorbent is split in two from a center thereof to one longitudinal end thereof in a widthwise direction; a non-split section of the absorbent is located at a substantially center of the rear abdominal region, and the split portions of the absorbent are provided at an intermediate section between the leg openings in the front abdominal region and on both sides (more specifically, the right and left sides of cemented section) of and along the cemented section.
   Since, the cemented section is located at the center of the front abdominal region, and the split portions of the absorbent located in the cemented section are located on both sides of the cemented section. Hence, by means of cementing operation required for forming a brief-type diaper from the pre-form blank for a diaper, the absorbent is pulled, whereby a bulge is formed from the absorbent in the crotch section in the rear abdominal region. By means of the bulge, a penis can be accommodated and held, thereby diminishing the chance of leakage of urine at the time of passage of urine.
(8) The brief-type diaper according to (1) or (2), wherein the absorbent is provided so as to spread across the front and rear abdominal regions by way of the crotch section, thereby covering the cemented section.
   According to the invention (8), the absorbent is located so as to spread across the front and rear abdominal regions via the crotch section, thereby covering the cemented section. Therefore, the absorbent is also present on the upper surface of the cemented section, thereby eliminating the fear of seepage of body fluids, such as urine, from the cemented section.
(9) The brief-type diaper according to any one of (6) through (8), wherein a leakage prevention wall is provided along longitudinal edges of the absorbent over the entire length thereof.
   According to the invention (9), a leakage prevention wall is provided along longitudinal edges of the absorbent over the entire length thereof. Therefore, a leakage prevention wall is formed along the edges of the leg openings from the longitudinal center over the entire length thereof, thereby preventing further leakage of excretions such as urine.
(10) The pre-form blank for a brief-type diaper according to (3) or (5), wherein the elastic member is provided along an upper edge of the pre-girth section in an extended manner.
   According to the invention (10), the elastic member is provided along an upper edge of the pre-girth section in an extended manner. When the pre-form blank for a diaper is folded and superimposed side edge portions are cemented together, to thereby form a waist opening, the elastic member is subjected to contraction, thereby rendering the girth region elastic. Therefore, the diaper conforms to the skin of the wearer when the diaper is worn. The elastic members are preferably made such that the degree of elongation and stress change from the waist region to the girth region. As a result, the degree of fastening changes from the waist region to the girth region, thereby causing the diaper to conform much better to the figure of the wearer's body. In the invention, the term "waist region" means a portion of the diaper which conforms to a position on the body above the hip bone.
(11) The pre-form blank for a brief-type diaper according to (4), wherein the elastic member is provided along an outer brim of the pre-girth section in a stretched manner.
   According to the invention (11), the elastic member is provided along an outer brim of the pre-girth section of the pre-form blank for a brief-type diaper in a stretched manner. Therefore, when the pre-form blank for a brief-type diaper is folded and the lower edge portions of a chord of a superimposed semicircular pattern are cemented together, to thereby form a brief-type diaper, the elastic member contracts, thereby rendering the girth section elastic. Therefore, the diaper conforms to the skin of the wearer when the diaper is worn. The elastic members are preferably made such that the degree of elongation and stress change from the waist region to the girth region. As a result, the degree of fastening changes from the waist region to the girth region, thereby causing the diaper to conform much better to the figure of the wearer's body.
(12) The pre-form blank for a brief-type diaper according to (3) or (4), wherein the elastic member is provided along peripheral edges of the pre-leg openings in a stretched manner.
   According to the invention (12), the elastic member is provided along peripheral edges of the pre-leg openings in a stretched manner. Therefore, when the pre-form blank is folded and the superimposed side edge portions of the pre-girth section and the lower edge portions of the pre-crotch section are cemented together, to thereby form a brief-type diaper, the elastic member contracts, thereby rendering the leg opening sections elastic. Therefore, the diaper conforms to the skin of the wearer when the diaper is worn.
(13) The pre-form blank for a brief-type diaper according to (5), wherein the elastic member is provided along a peripheral edge of the peak-shaped notch across the pre-girth section and the pre-crotch section in an elongated state.
   According to the invention (13), the elastic member of pre-form blank for a brief-type diaper is provided along a peripheral edge of the peak-shaped notch across the pre-girth section and the pre-crotch section in an elongated state. Therefore, when the pre-form blank is folded and the superimposed side edge portions and lower edge portions of the pre-girth section and the lower edge portions of the pre-crotch section are cemented together, to thereby form a brief-type diaper, the elastic member contracts, thereby rendering the leg opening sections elastic. Therefore, the diaper conforms to the skin of the wearer when the diaper is worn.

### BRIEF DESCRIPTION OF THE DRAWINGS

Fig. 1 is a partially fragmented developed plan view showing a developed state of a brief-type diaper according to a first embodiment of the invention.
Fig. 2 is a schematic perspective view showing the brief-type diaper showing the first embodiment of the invention when viewed from the front.
Fig. 3 is a schematic perspective view showing the brief-type diaper of the first embodiment of the invention when viewed from the rear.
Fig. 4 is a schematic perspective view of the brief-type diaper of the first embodiment when viewed from the side.
Fig. 5 is a schematic perspective view of a modification of the brief-type diaper shown in Fig. 2.
Fig. 6 is a descriptive view showing a case where a leakage prevention wall is provided on the brief-type diaper of the first embodiment shown in Fig. 1;
Fig. 7 is a plan view of a brief-type diaper obtained as a result of formation of the diaper shown in Fig. 6.
Fig. 8 is a partially fragmented developed plan view showing a developed state of a brief-type diaper according to a second embodiment of the invention.
Fig. 9 is a schematic perspective view showing the brief-type diaper of the second embodiment of the invention when viewed from the front.
Fig. 10 is a partially fragmented developed plan view showing a developed state of a brief-type diaper according to a third embodiment of the invention.
Fig. 11 is a schematic perspective view showing the brief-type diaper of the third embodiment of the invention when viewed from the front.
Fig. 12 is a developed plan view of a conventional brief-type diaper.

### DESCRIPTION OF THE PREFERRED EMBODIMENT

Embodiments of the invention will be described in detail hereinbelow by reference to the drawings.

### [First Embodiment]

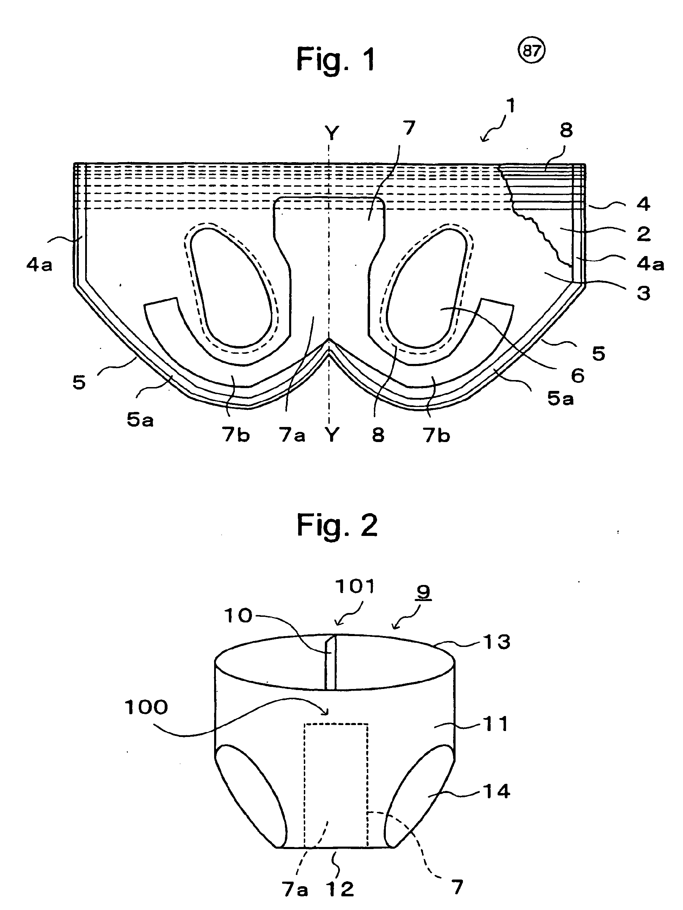

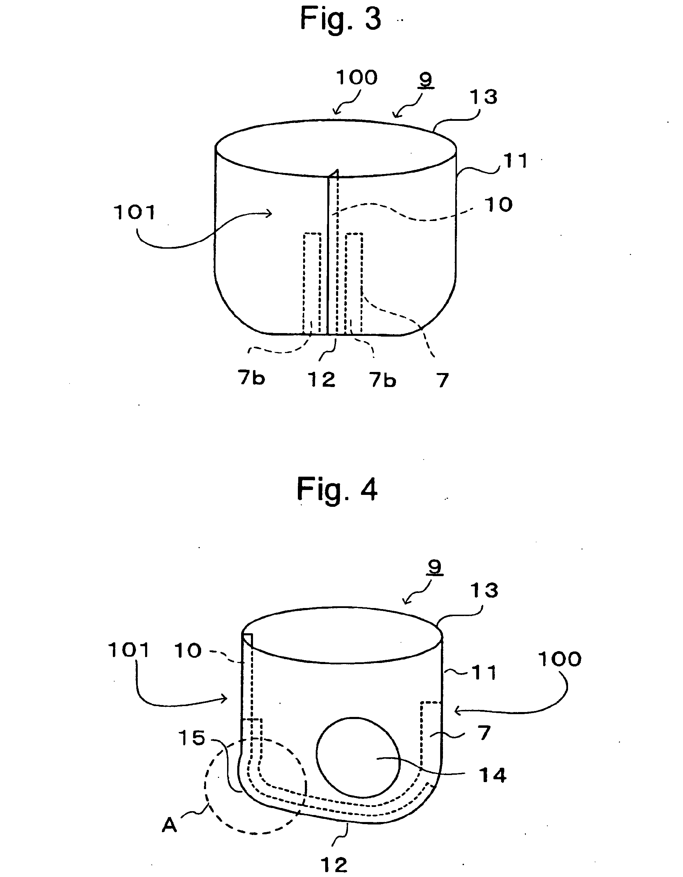

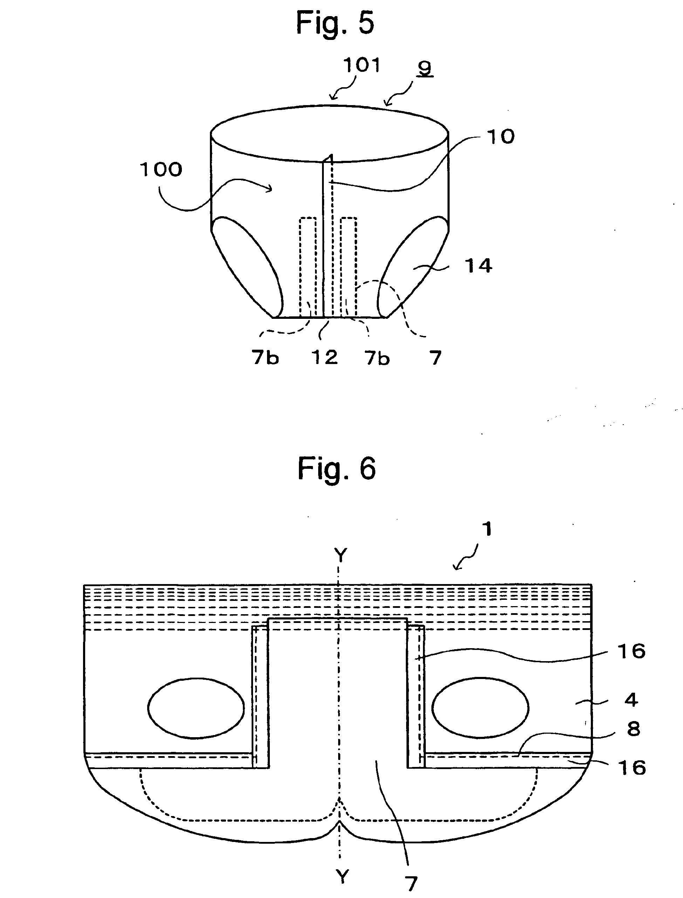

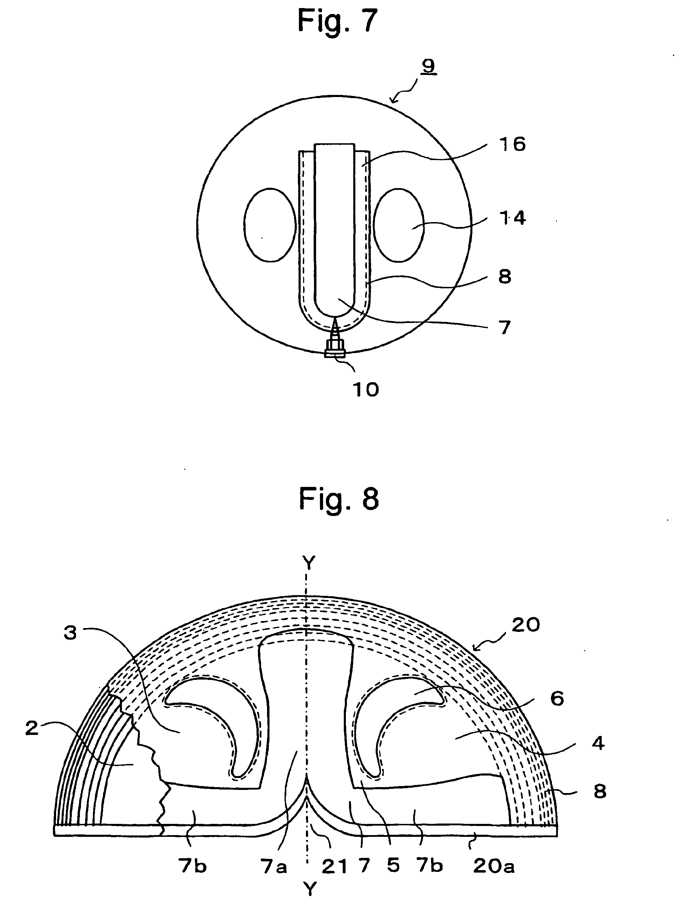

Fig. 1 is a partially fragmented developed plan view showing a developed state of a brief-type diaper according to a first embodiment of the invention, and Fig. 2 is a schematic perspective view showing the brief-type diaperof the first embodiment of the invention when viewed from the front, and Fig. 3 is a schematic perspective view showing the brief-type diaper of the first embodiment of the invention when viewed from the rear, and Fig. 4 is a schematic perspective view showing the brief-type diaper of the first embodiment of the invention when viewed from the side, and Fig. 5 is a schematic perspective view of a modification of the brief-type diaper shown in Fig. 2, and Fig. 6 is a descriptive view showing a case where a leakage prevention wall is provided on the brief-type diaper of the first embodiment shown in Fig. 1, and Fig. 7 is a plan view of a brief-type diaper obtained as a result of formation of the diaper shown in Fig. 6;

Fig. 1 shows a brief-type diaper according to a first embodiment of the invention while being developed. In the drawing, reference numeral 1 designates a pre-form blank for a brief-type diaper. The pre-form blank for a brief-type diaper 1 is constituted of an outer sheet 2 comprises an exterior and an inner sheet 3 being brought into contact with wearer's skin. The pre-form blank for a brief-type diaper 1 comprises a substantially-swath pre-girth section 4 and pre-crotch sections 5 formed in the shape of twin peaks. The pre-crotch sections 5 are formed into peaks which are symmetrical about the Y-Y axis of symmetry of the pre-form blank for a brief-type diaper 1, and bulge from entire lower edge of the pre-girth section 4. A pair of pre-leg openings 6 are formed in the form of substantially oval holes at respective positions on the pre-crotch sections 5 the positions being symmetrical with respect to the Y-Y axis of symmetry of the pre-form blank 1, so as to straddle the pre-girth section 4.

Reference numeral 7 designates an absorbent, and the absorbent 7 comprises a liquid-impervious back sheet, a liquid-pervious top sheet, and an absorbent core interposed between the sheets. One longitudinal end of the absorbent core 7 is split in two in a transverse direction of the absorbent core 7 from the center section thereof. A non-split section 7a of the absorbent 7 is provided at the center of the pre-girth section 4 such that a longitudinal direction of the absorbent 7 is oriented vertically. Split sections 7b are provided along respective lower edge portions 5a of the pre-crotch section 5.

The inner sheet 3 is bonded to the upper surface of the outer sheet 2 (more specifically, body surface side of the wearer), and the absorbent 7 is laminated and bonded onto the upper surface of the inner sheet 3 (more specifically, body surface side of the wearer). An elastic member 8 is horizontally provided in a stretched manner along the upper end edge of the pre-girth section 4 and in the vicinity of the peripheral edges of the respective pre-leg openings 6. In this case, the elastic member 8 to be provided at the pre-girth section 4 is preferably made such that the degree of elongation changes from the waist region to the girth region. Thus, the degree of fastening changes from the waist region to the girth region, thereby causing the diaper to conform much better to the figure of the wearer's body. In other words, providing an appropriate fit around the waist by tightening the brief-type diaper to keep the diaper from falling off the waist and fit the girth region at ease. The way to change the fitness around the waist and girth section may also be achieved by selecting the number of elastic members, intervals at which the elastic members 8 are to be stretched, the thickness of the elastic members, and materials of the elastic members, as required, rather than changing the degree of its elongation. In addition, settings on degree of elongation and stress of elastic member 8 can be same.

Throughout the specification, the horizontal direction signifies an X direction, provided that the Y-Y axis of symmetry of the pre-form blank for a brief-type diaper for a brief-type diaper 1 is taken as the Y direction and a direction orthogonal to the Y direction is taken as an X direction. Further, the term "waist region" means a part of the diaper which conforms to a position on the body above the hip bone upon wearing them. Further, the term "girth section" means a part of the diaper which conforms to the area of the body ranging from the hip bone to the crotch. Moreover, the term "front abdominal region" means a portion of a brief-type diaper facing the abdomen of the body. The term "rear abdominal region" means a portion of the diaper facing the back of the body.

When a brief-type diaper 9 shown in Fig. 2 is formed from the pre-form blank for a brief-type diaper 1, the non-split section 7a of the absorbent 7 comes to the center of the pre-girth section 4 on the upper surface of the pre-form blank for a brief-type diaper 1 (more specifically, a body surface side of the wearer) such that the longitudinal direction of absorbent 7 is oriented in the vertical direction. The split sections 7b are provided along the lower edge portions 5a of the pre-crotch sections 5 in lateral direction. The pre-girth section 4 and the pre-crotch sections 5 are folded horizontally along the axis (Y-Y) of symmetry. The pre-girth section 4 and the pre-crotch sections 5 are superimposed one on the other. The side edge portions 4a of the superimposed pre-girth section 4 are cemented together, and the lower edge portions 5a of the pre-crotch sections 5 are cemented together, thereby forming a waist opening 13. A cementing method to be employed in this embodiment includes thermal fusing, ultrasonic fusing, and bonding involving use of an adhesive such as a hot melt adhesive.

Fig. 2 through Fig. 4, shows the thus-formed brief-type diaper 9. A cemented section 10 is formed in the brief-type diaper 9 so as to extend straight from the waist opening 13 to the crotch section 12 in the longitudinal direction along the center section of the rear abdominal region 101.

More specifically, as shown in Fig. 4, the cemented section 10 passes through the crotch section 12 so as to extended from of the waist opening section 13 of the rear abdominal region 101 side, thereby reaching a boundary between the crotch section 12 and the girth section 11 in the front abdominal region 100. The absorbent 7 is arranged so as to spread across the front and rear abdominal regions, 100, 101, via the crotch section 12. As shown in Figs. 2 and 3, the non-split section 7a of the absorbent 7 is placed in the girth section 11 and the crotch section 12 within the front abdominal region 100 and the split sections 7b of the absorbent 7 are placed along the right and left sides of the cemented section 10 across the girth section 11 and the crotch section 12 within the rear abdominal region 101. Thus, the absorbent 7 is split, and the crotch section 12 is formed in a curved shape. Hence, the outer sheet and the inner sheet are pushed by the absorbent 7 in the crotch section 12 of the rear abdominal region 101 (within the areas A encircled by dotted lines in Fig. 4), thereby forming a bulging section 15.

In the brief-type diaper 9, the rigid cemented section 10 extends in the longitudinal direction along the center section of the rear abdominal region 101 so as to avoid the leg openings 14. Hence, the wearer does not have any unusual feeling around the girth or the legs.

Also, the cemented section 10 is located at the center of the rear abdominal region 101 spaced apart from lateral portions of waist opening 13. Therefore, when the wearer wears the brief-type diaper 9, the cemented section 10 does not hinder the wearer from wearing the diaper 9, allowing he/she to readily pull up while pinching the right and left side of waist opening 13 to broaden the opening. Here, the cemented section 10 does not catch any part of the wearer's body.

Further, the bulging section 15 arises in the crotch section 12 of the rear abdominal section 101, whereby a space is defined between the absorbent 7 and the wearer's skin. Since the wearer's excretions such as fecal matter are housed in the space, the degree to which the excretions adhere to the wearer's skin can be diminished. Further, the non-split section 7a of absorbent 7 at the front abdominal region 100 assumes a flat surface which is likely to absorb body fluids such as urine. Thus, an absorptive structure ideal for urine and another absorptive structure ideal for fecal matter can be established.

As shown in Fig. 5, the cemented section 10 may be arranged in the front abdominal region 100. In this case, the split sections 7b of the absorbent 7 are arranged in the front abdominal region 100. Hence, the bulging section is formed in the crotch section 12 of the front abdominal region 100 (see Fig. 4). As a result, the penis is accommodated in the bulging section, thereby lessening the chance of leakage of urine, which would otherwise arise when urine is passed.

Alternatively, a leakage prevention wall 16 may be provided beforehand on both longitudinal side edges of the absorbent 7 throughout its length. As shown in Fig. 6, if the leakage prevention wall 16 is provided along both longitudinal side edges of the absorbent 7, the leakage prevention wall 16 will be provided along a boundary between the absorbent 7 and the pre-girth section 4 of the pre-from blank for a diaper 1. If it is formed into the brief-type diaper 9, the extremity of the leakage prevention wall 16 is closed at the cemented section 10. When viewed from above, the leakage prevention wall 16 assumes a substantially-U-shaped pattern such as that shown in Fig. 7. The elastic member 8 is provided along a side edge of the leakage prevention wall 16 in an elongated state and when it is formed into the brief-type diaper 9, causes elastic member 8 to contract and raise leakage prevention wall 16. Provision of the leakage prevention wall 16 yields the effect of the ability to prevent leakage of body fluids such as urine from the absorbent 7.

The outer sheet 2 and the inner sheet 3, which are members for constituting the brief-type diaper 9, are preferably made of gas-permeable nonwoven fabric cloth, more preferably of hydrophobic nonwoven fabric cloth. For example, the nonwoven fabric cloth includes spun bond nonwoven fabric cloth made of polypropylene or the like; through-air nonwoven fabric cloth made of fibers, such as polyethylene fibers, polypropylene fibers, polyethylene terephthalate fibers, or the like; and spun lace nonwoven fabric cloth. In addition, there may also be employed a complex sheet formed from nonwoven fabric ctoth-which includes elastomer or copolymer and possesses flexibility and an extensible characteristic-and a flexible film. Moreover, a liquid-impervious plastic film may be sandwiched between the outer sheet 2 and the inner sheet 3.

The absorbent 7 is formed from a rectangular or guitar-shaped absorbent core on the liquid-impervious back sheet, liquid-pervious top sheet as well as between these sheets such that each longitudinal end of the core is split from a longitudinal center in a transverse direction of the core. The liquid-impervious back sheet is nonwoven fabric cloth which has been subjected to water repellent finishing, a moisture-permeable plastic film having minute pores, or a layered product consisting of the fabric cloth and the plastic film. Nonwoven fabric cloth is usually used for the liquid-pervious top sheet. The absorbent core is preferably a core made by combined use of defibrated pulp and water-absorptive polymer. In addition, a core-which is formed from a mixture of cellulose fibers, thermoplastic resin, heat-fusible fibers, and the like and which has been subjected to heat treatment-is also preferable. In the case of an absorbent into which several layers are stacked, the water-absorptive polymer may be located in an upper layer, an intermediate layer, or a lower layer. Alternatively, the polymer may be uniformly mixed with pulp. The water-absorptive polymer preferably has the capability of absorbing liquid which is 20 times or more the weight of the polymer, as well as the property of gelation. Preferable examples of water-absorptive polymer include starch-acrylic acid (acrylate) graft copolymer, saponificated starch-acrylonitrile copolymer, crosslinked sodium carboxymethyl cellulose, and acrylic acid (acrylate) polymer.

The elastic members 8 are not limited to any particular material but are formed from any material, so long as the material possesses elasticity; for example, rubber yarn made of natural/synthetic rubber or polyurethane, a ribbon-shaped elastic body, heat-shrinkable material, and water-absorptive shrinkable fibers.

### [Second Embodiment]

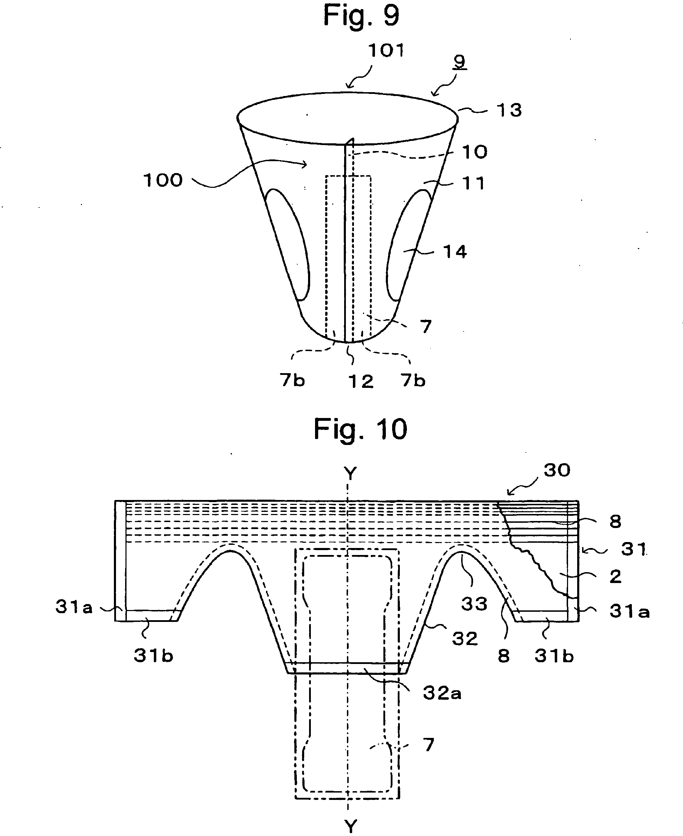

Fig. 8 provides a partially fragmented developed plan view of a brief-type diaper according to a second embodiment of the invention. Fig. 9 provides a front schematic perspective view of a brief-type diaper according to a second embodiment of the invention. In the embodiment, those constituent elements which are the same as those described in connection with the first embodiment are assigned the same reference numerals, and their explanations are omitted.

Fig. 8 provides a partially fragmented developed plan view of a brief-type diaper according to a second embodiment of the invention. Reference numeral 20 designates a pre-form blank for a brief-type diaper of the second embodiment. As in the case of the first embodiment, the pre-form blank 20 is comprised of the liquid-impervious outer sheet 2 which constitutes exterior and the liquid-pervious inner sheet 3 which is brought into contact with the wearer's skin, assumes a substantially-semicircular outer shape, being provided in a single cloth, wherein a substantially-V-shaped indentation 21 is formed at a substantial center of the chord of a substantially-semicircular pattern. The pre-girth section 4 and the pre-crotch section 5 are present as an arc portion of the substantially-semicircular pattern. The elastic member 8 is provided in a stretched state in the pre-girth section 4 located outside the substantially-semicircular arc. The pre-leg openings 6 are formed in a pair form of substantially-crescent-shaped holes at respective positions on the pre-girth section 4 so as to extend from the pre-girth section 4 to the pre-crotch section 5, the positions being symmetrical with respect to the axis (Y-Y) of symmetry of the pre-form blank for a brief-type diaper 20. The elastic member 8 is provided in a stretched state horizontally along the upper edge of the pre-girth section 4 and in the vicinity of peripheral edges of the pre-leg openings 6. The geometry of the pre-leg opening 6 is not limited to a substantial crescent shape but may be circular or semi-circular or may take the form of a simple cut line (slit).

The pre-form blank for a brief-type diaper 20 of the embodiment has the V-shaped notch 21 at substantially the center of the chord of the substantially-semicircular pattern. However, the notch is not limited to this notch. For instance, the notch may be embodied as a slit-like notch extending from the center of the chord to the axis of symmetry Y-Y, or a U-shaped-like notch having an acute or obtuse head.

When the brief-type diaper 9 of Fig. 9 is formed from the pre-form blank for a brief-type diaper 20, the non-split section 7a of the absorbent 7 that has been split into two from the longitudinal center thereof to one end with respect to the transverse direction thereof is arranged along the axis of symmetry of the pre-form blank for a brief-type diaper 20 such that the longitudinal direction of the absorbent 7 is oriented vertically at the upper surface of inner sheet 3 (Body side surface of the wearer). Each respective split sections 7b are arranged along edge portions 20a of the chord of the substantially-semicircular pattern. The pre-form blank for a brief-type diaper 20 having the absorbent 7 provided thereon is folded horizontally along the axis of symmetry Y-Y, thereby cementing together the superimposed edges 20a of the chord of the substantially-semicircular pattern, forming the waist opening 13.

Fig. 9 shows the thus-formed brief-type diaper 9. The brief-type diaper 9 has such a shape that the girth section 11 is tapered toward the crotch section 12. The cemented section 10 is formed in the front abdominal region 100 from the girth opening 13 to the crotch section 12. The absorbent 7 is provided so as to extend across the front and rear abdominal regions 101 via the crotch section 12. As being said, the split sections 7b of the absorbent 7are placed on both sides of the cemented section 10 so as to extend from the girth section 11 to the crotch section 12 of the front abdominal region 100, and the non-split section 7a of the absorbent 7 is placed in the center area of the rear abdominal region 101 (see Fig.2 through Fig.4). Thus, the absorbent 7 is split, and the crotch section 12 is formed in a curved shape by means of cementing operation. Although not-illustrated, as previously described, that the outer sheet and the inner sheet in the crotch section 12 of the front abdominal region 100 are pushed by the absorbent 7, thereby forming the bulging section in the crotch section.

However, the cemented section 10 may be formed on the rear abdominal region 101. In this case, bulging section is formed in the crotch section 12 of the rear abdominal region 101.

### [Third Embodiment]

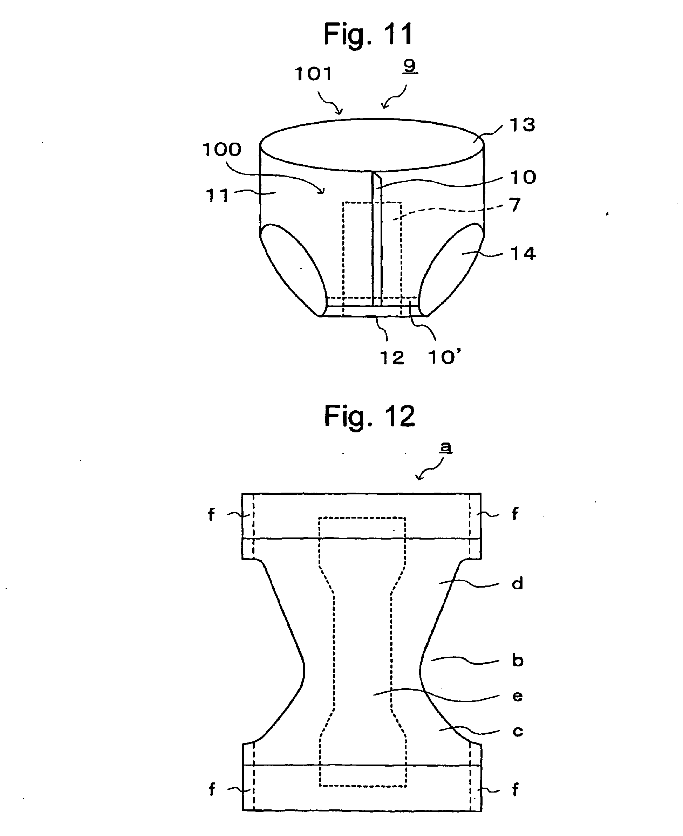

Fig. 10 provides a partially fragmented developed plan view showing a developed state of a brief-type diaper according to a third embodiment of the invention. Fig. 11 is a schematic perspective view showing the brief-type diaper of the third embodiment of the invention when viewed from the front. In the embodiment, those constituent elements which are the same as those described in connection with the first embodiment are assigned the same reference numerals, and their explanations are omitted.

Fig. 10 provides a partially fragmented developed plan view showing a developed state of a brief-type diaper according to a third embodiment of the invention. Reference numeral 30 designates a pre-form blank for a brief-type diaper of brief-type diaper according to the third embodiment, is comprised of the liquid-impervious outer sheet 2 forming an exterior as being similar to the first embodiment and the liquid-pervious inner sheet 3 which is brought in contact with wearer's skin. The pre-form blank for a brief-type diaper 30 assumes a substantially-rectangular outer shape, is a single cloth having a substantially-trapezoidal protrusion whose lower central edge protrudes, is formed from a substantially-rectangular pre-girth section 31 and a trapezoidal pre-crotch section 32 which extends from a lower central edge of the pre-girth section 31. The elastic member 8 is horizontally arranged in a stretched state along the upper edge of the pre-girth section 31. A peak-shaped notch 33 is formed on either side of the pre-crotch section 32 along the lower edge of the pre-girth section 31. The elastic member 8 is provided in a stretched state along the peripheral edge of the notch 33.

When a brief-type diaper 9 shown in Fig. 11 is formed from the pre-form blank for a brief-type diaper 30, wherein an essentially upper longitudinal half of the absorbent 7 is arranged at the upper surface of the inner sheet 3 (lateral body side of the wearer), and an intermediate section of the pre-girth section 31 of the pre-form blank for a brief-type diaper 30, is folded horizontally along the axis (Y-Y) of symmetry. Side edge portions 31a of the superimposed pre-girth section 31 are cemented together, thereby forming the waist opening 13. A lower edge portions 32a of the pre-crotch section 32 and a lower edge portion 31b of the pre-girth section 31 are cemented together, thereby forming the leg openings 14.

Fig. 11 shows the thus-formed brief-type diaper 9. In the case of the brief-type diaper 9, a cemented section 10' formed by cementing together the lower edge portion 32a of the pre-crotch section 32 and the lower edge portion 31b of the pre-girth section 31 extends laterally (horizontally) across the crotch section 12 of the front abdominal region 100. Moreover, the cemented section 10 formed by cementing together the side edge portions 31 a extends from the waist opening 13 straight across the center of the front abdominal region 100 vertically (longitudinal direction) and reaches the cemented section 10' of the crotch section 12. Since the absorbent 7 remains unsplit, no bulging arises in the crotch section 12 of the front abdominal region 100 or the crotch section 12 of the rear abdominal region 101. Thus, a silhouette of the diaper obtained when the wearer wears the diaper is improved. Moreover, the absorbent 7 covers the cemented section 10, and hence seepage of body fluids, such as urine, from the cemented section can be prevented.

However, this cemented section 10 may be formed on the rear abdominal region 101. Although not illustrated, a cemented section 10' formed by cementing together the lower edge portion 32a of the pre-crotch section 32 and the lower edge portion 31b of the pre-girth section 31 extends laterally (horizontally) across the crotch section 12 of the rear abdominal region 101. Moreover, the cemented section 10 formed by cementing together the side edge portions 31a of the pre-girth region 31, which extends from the girth opening 13 straight across the center of the front abdominal region 100 vertically (longitudinal direction), thus reaching the cemented section 10' of the crotch section 12.

In any one of the first through third embodiments, the absorbent 7 is placed on the upper surface of the inner sheet 3 (Body surface side of the wearer), is cemented together on the inner sheet 3. However, the invention is not limited to such a cemented structure. The absorbent may be sandwiched between and cemented to the outer sheet 2 and the inner sheet 3 (not shown). The inner sheet to be used at this time is preferably liquid-pervious nonwoven fabric cloth.

The inner sheet 3 may be substantially identical in size and shape with the outer sheet 2 and have an opening section, which essentially corresponds to the absorbent 7, in substantially the center thereof (not shown). In this case, the inner sheet is preferably hydrophobic nonwoven fabric cloth. As described, when an opening section is formed in the inner sheet 3, the absorbent 7 becomes exposed from the inner sheet 3. Therefore, such a structure is preferable as a leakage prevention wall 16, being provided along both longitudinal ends of the absorbent 7 over its entire length, when the leakage prevention wall 16 is raised from inner sheet 3.

However, the brief-type diaper of the invention is not limited to the embodiments described above and may be modified within the scope of the invention, as required. Further, the shape and material of the brief-type diaper are not limited to those mentioned described in connection with the embodiments.

According to the invention, a cemented section extends from the waist opening to the crotch section while avoiding leg openings while the wearer does not have any unusual feeling around the girth or the legs wherein, the cemented section is located at the region spaced apart from both right and left ends of the waist opening. Therefore, when the right and left side of waist opening are pinched to be broadened, the cemented section does not hinder the wearer from wearing the diaper. Hence, wearing of the diaper is facilitated.

When two split sections into which a part of the absorbent is split are provided along the cemented section, a bulge arises in the absorbent within the crotch section of the cemented section. Space formed between the absorbent and the skin of the wearer, to house excretions such as fecal matter, can reduce the chance of the excretions adhering to the wearer's skin.

## Claims

1. A brief-type diaper having an outer sheet (2) constituting an exterior, an inner sheet (3) which comes into contact with a wearer, an absorbent (7) for absorbing body fluids, an elastic member (8) which is provided on either the inner sheet (3) or the outer sheet (2) or on both sheets and which causes portions of the outer and inner sheets to contract and extend, and a pair of leg openings (6) through which the wearer's legs penetrate, the diaper comprising:
a cemented section (10) which extends from a waist opening (13) to a crotch section (12) while avoiding the leg openings in a front or rear abdominal region of the brief-type diaper.

2. The brief-type diaper according to claim 1, wherein the cemented section (10) is a continuous fiber which extends across a substantial center section of the front or rear abdominal region in a direction in which the brief-type diaper is to be worn.

3. A pre-form blank (1) for a brief-type diaper which is formed from a substantially-W-shaped single cloth made by cutting one side of a rectangle in the form of twin peaks and which has a pre-girth section (4) to act as a girth section, pre-leg opening sections (6) to act as leg opening sections, and a pre-crotch section (5) to act as a crotch section, the pre-form blank further comprising:
the pre-girth section (4) being present in the form of a substantially-rectangular portion, wherein an elastic member (8) is provided in the pre-girth section (4) in a stretched manner;
the pre-leg openings (6) being present in the form of a pair of holes and at positions which are symmetrical about the axis of symmetry of the pre-form blank for brief-type diaper; and
the pre-crotch section (5) being present in the form of twin peaks extending from a lower edge of the substantially-rectangular pre-girth section.

4. A pre-form blank (20) for a brief-type diaper which is formed from a single cloth, the cloth assuming a substantially-semicircular shape and having a notch section (21) at substantially the center of a chord of the semicircular pattern, and which has a pre-girth section (4) to act as a girth section, pre-leg opening sections (6) to act as leg opening sections, and a pre-crotch section (5) to act as a crotch section, the pre-form blank (20) further comprising:
the pre-girth section (4) and the pre-crotch section (5) being located as a chord of the semicircular pattern, in which an elastic member (8) is provided in the pre-girth section (4) in an elongated state; and
the pre-leg openings (6) being present in the form of holes and at positions which are symmetrical about the axis of symmetry (Y-Y) of the pre-form blank for a brief-type diaper.

5. A pre-form blank (30) for a brief-type diaper which is formed from a single cloth, the cloth assuming a substantially-rectangular shape and having a substantially-trapezoidal protrusion projecting from a center of a lower edge of the rectangular edge, and which has a pre-girth section (31) to act as a girth section, pre-leg opening sections to act as leg opening sections, and a pre-crotch section (32) to act as a crotch section, the pre-form blank (30) further comprising:
the pre-girth section (31) being present in the form of a substantial-rectangular portion, wherein an elastic member (8) is provided in the pre-girth section (31) in a stretched manner;
the pre-leg openings being present in the form of a peak-shaped notch (33) in the pre-girth section (31) and the pre-crotch section (32) and straddling the substantially-trapezoidal protrusion; and
the pre-crotch section (32) being present in the form of a substantially-trapezoidal protrusion extending from a center of a lower edge of the substantially-rectangular pre-girth section (31).

6. The brief-type diaper according to claim 1 or 2, wherein the absorbent (7) is split in two from a center thereof to one longitudinal end thereof in a widthwise direction; a non-split section (7a) of the absorbent is located at an intermediate section between the pair of leg openings (14) in the front abdominal region (100), and the split portions (7b) of the absorbent are provided at a substantial center of the rear abdominal region (101) and on both sides of and along the cemented section (10).

7. The brief-type diaper according to claim 1 or 2, wherein the absorbent (7) is split in two from a center thereof to one longitudinal end thereof in a widthwise direction; a non-split section (7a) of the absorbent is located at a substantial center of the rear abdominal region (101), and the split portions (7b) of the absorbent are provided at an intermediate section between the leg openings (14) in the front abdominal region (100) and on both sides of and along the cemented section (10).

8. The brief-type diaper according to claim 1 or 2, wherein the absorbent (7) is provided so as to spread across the front and rear abdominal regions (100,101) by way of the crotch section (12), thereby covering the cemented section (10).

9. The brief-type diaper according to any one of claims 6 through 8, wherein a leakage prevention wall (16) is provided along longitudinal edges of the absorbent (7) over the entire length thereof.

10. The pre-form blank for a brief-type diaper according to claim 3 or 5, wherein the elastic member (8) is provided along an upper edge of the pre-girth section (4) in an extended manner.

11. The pre-form blank for a brief-type diaper according to claim 4, wherein the elastic member (8) is provided along an outer brim of the pre-girth section (4) in a stretched manner.

12. The brief-type diaper according to claim 3 or 4, wherein the elastic member (8) is provided along peripheral edges of the pre-leg openings (6) in a stretched manner.

13. The brief-type diaper according to claim 5, wherein the elastic member (8) is provided along a peripheral edge of the peak-shaped notch (33) across the pre-girth section (31) and the pre-crotch section (32) in an elongated state.

## Patentansprüche

1. Hosenartige Windel mit einer eine Außenseite bildenden Außenlage (2), mit einer mit einem Träger in Kontakt kommenden Innenlage (3), mit einem Absorbens (7) zum Absorbieren von Körperflüssigkeiten, mit einem elastischen Element (8) an der Innenlage (3), der Außenlage (2) oder beiden Lagen, das Teile der Außen- und Innenlagen zum Zusammenziehen und zum Ausdehnen veranlasst, und mit einem Paar von Beinöffnungen (6), durch welche sich die Beine des Trägers erstrecken, wobei die Windel umfasst:
einen verklebten Abschnitt (10), der sich unter Umgehung der Beinöffnungen in einem vorderen oder hinteren Unterleibsbereich der hosenartigen Windel von einer Taillenöffnung (13) zu einem Schrittabschnitt (12) erstreckt.

2. Hosenartige Windel nach Anspruch 1, wobei der verklebte Abschnitt (10) eine Endlosfaser ist, die sich über einen wesentlichen mittleren Abschnitt des vorderen oder hinteren Unterleibsbereichs in einer Richtung erstreckt, in der die hosenartige Windel zu tragen ist.

3. Vorgeformter Zuschnitt (1) für eine hosenartige Windel, der aus einem einzelnen, im Wesentlichen W-förmigen Tuch gebildet ist, das durch Zuschneiden einer Seite eines Rechtecks in der Form eines Doppelgipfels hergestellt wird und einen als Umfangsabschnitt wirkenden Umfangs-Vorabschnitt (4), als Beinöffnungsabschnitte wirkende Beinöffnungs-Vorabschnitte (6) und einen als Schrittabschnitt wirkenden Schritt-Vorabschnitt (5) aufweist, wobei der vorgeformte Zuschnitt weiter umfasst:
den Umfangs-Vorabschnitt (4) in der Form eines im Wesentlichen rechteckigen Teils, wobei im Umfangs-Vorabschnitt (4) ein elastisches Element (8) im gedehnten Zustand vorgesehen ist;
die Beinöffnungs-Vorabschnitte (6) in der Form eines Paares von Löchern und an Stellen, die symmetrisch um die Symmetrieachse des vorgeformten Zuschnitts für eine hosenartige Windel liegen; und
den Schritt-Vorabschnitt (5) in der Form eines Doppelgipfels, der sich von einem unteren Rand des im Wesentlichen rechteckigen Umfangs-Vorabschnitts aus erstreckt.

4. Vorgeformter Zuschnitt (20) für eine hosenartige Windel, der aus einem einzelnen Tuch besteht, wobei das Tuch im Wesentlichen eine Halbkreisform und einen Kerbabschnitt (21) im Wesentlichen in der Mitte einer Sehne der Halbkreisform aufweist, und der einen als Umfangsabschnitt wirkenden Umfangs-Vorabschnitt (4), als Beinöffnungsabschnitte wirkende Beinöffnungs-Vorabschnitte (6) und einen als Schrittabschnitt wirkenden Schritt-Vorabschnitt (5) aufweist, wobei der vorgeformte Zuschnitt (20) weiter umfasst:
den Umfangs-Vorabschnitt (4) und den Schritt-Vorabschnitt (5) als Sehne der Halbkreisform, wobei im Umfangs-Vorabschnitt (4) ein elastisches Element (8) im gedehnten Zustand vorgesehen ist; und
die Beinöffnungs-Vorabschnitte (6) in der Form von Löchern an Stellen, die symmetrisch um die Symmetrieachse (Y-Y) des vorgeformten Zuschnitts für eine hosenartige Windel liegen.

5. Vorgeformter Zuschnitt (30) für eine hosenartige Windel, der aus einem einzelnen Tuch besteht, wobei das Tuch im Wesentlichen eine Rechteckform und einen im Wesentlichen trapezförmigen, aus einer Mitte eines unteren Randes der Rechteckskante hervorstehenden Vorsprung aufweist, und der einen als Umfangsabschnitt wirkenden Umfangs-Vorabschnitt (31), als Beinöffnungsabschnitte wirkende Beinöffnungs-Vorabschnitte und einen als Schrittabschnitt wirkenden Schritt-Vorabschnitt (32) aufweist, wobei der vorgeformte Zuschnitt (30) weiter umfasst:
den Umfangs-Vorabschnitt (31) in der Form eines im Wesentlichen rechteckigen Teils, wobei im Umfangs-Vorabschnitt (31) ein elastisches Element (8) im gedehnten Zustand vorgesehen ist;
die Beinöffnungs-Vorabschnitte in der Form einer gipfelförmigen Kerbe (33) im Umfangs-Vorabschnitt (31) und im Schritt-Vorabschnitt (32), die den im Wesentlichen trapezförmigen Vorsprung überspreizt; und
den Schritt-Vorabschnitt (32) in der Form eines im Wesentlichen trapezförmigen Vorsprungs, der sich von einer Mitte eines unteren Randes des im Wesentlichen rechteckförmigen Umfangs-Vorabschnitts (31) aus erstreckt.

6. Hosenartige Windel nach Anspruch 1 oder 2, wobei das Absorbens (7) von einer Mitte desselben bis zu einem Längsende desselben in Breitenrichtung in zwei Teile aufgeteilt ist; wobei ein nicht aufgeteilter Abschnitt (7a) des Absorbens in einem Mittelabschnitt zwischen dem Paar von Beinöffnungen (14) im vorderen Unterleibsbereich (100) liegt; und wobei die aufgeteilten Abschnitte (7b) des Absorbens im Wesentlichen in der Mitte des hinteren Unterleibsbereichs (101) vorgesehen sind und beidseitig längs des verklebten Abschnitts (10) liegen.

7. Hosenartige Windel nach Anspruch 1 oder 2, wobei das Absorbens (7) von einer Mitte desselben bis zu einem Längsende desselben in Breitenrichtung in zwei Teile aufgeteilt ist; wobei ein nicht aufgeteilter Abschnitt (7a) des Absorbens in einem in einem im Wesentlichen mittleren Abschnitt des hinteren Unterleibsbereichs (101) liegt; und wobei die aufgeteilten Abschnitte (7b) des Absorbens in einem Mittelabschnitt zwischen den Beinöffnungen (14) im vorderen Unterleibsbereich (100) vorgesehen sind und beidseitig längs des verklebten Abschnitts (10) liegen.

8. Hosenartige Windel nach Anspruch 1 oder 2, wobei das Absorbens (7) dazu vorgesehen ist, um sich über den Schrittabschnitt (12) über die vorderen und hinteren Unterleibsabschnitte (100, 101) zu erstrecken und **dadurch** den verklebten Abschnitt (10) abzudecken.

9. Hosenartige Windel nach einem der Ansprüche 6 bis 8, wobei entlang von Längsrändern des Absorbens (7) über dessen ganze Länge eine Austrittschutzwand (16) vorgesehen ist.

10. Vorgeformter Zuschnitt für eine hosenartige Windel nach Anspruch 3 oder 5, wobei das elastische Element (8) im gedehnten Zustand entlang einem oberen Rand des Umfangs-Vorabschnitts (4) vorgesehen ist.

11. Vorgeformter Zuschnitt für eine hosenartige Windel nach Anspruch 4, wobei das elastische Element (8) im gedehnten Zustand entlang einem Außenrand des Umfangs-Vorabschnitts (4) vorgesehen ist.

12. Hosenartige Windel nach Anspruch 3 oder 4, wobei das elastische Element (8) im gedehnten Zustand entlang Umfangsrändern der Beinöffnungs-Vorabschnitte (6) vorgesehen ist.

13. Hosenartige Windel nach Anspruch 5, wobei das elastische Element (8) im gedehnten Zustand entlang einem Umfangsrand der gipfelförmigen Kerbe (33) über den Umfangs-Vorabschnitt (31) und den Schritt-Vorabschnitt (32) vorgesehen ist.

## Revendications

1. Couche-culotte de type culotte ayant une feuille extérieure (2) constituant un extérieur, une feuille intérieure (3) qui entre en contact avec un utilisateur, un absorbant (7) destiné à absorber des liquides organiques, un élément élastique (8) qui est mis en oeuvre sur soit la feuille intérieure (3) soit la feuille extérieure (2) soit sur les deux feuilles et qui entraîne des parties des feuilles extérieure et intérieure à se contracter et à s'étendre, et une paire d'ouvertures pour les jambes (6) au travers desquelles les jambes de l'utilisateur pénètrent, la couche-culotte comportant :
une section collée (10) qui s'étend depuis une ouverture pour la taille (13) jusqu'à une section entrejambe (12) tout en évitant les ouvertures pour les jambes dans une région abdominale avant ou arrière de la couche-culotte de type culotte.

2. Couche-culotte de type culotte selon la revendication 1, dans laquelle la section collée (10) est une fibre continue qui s'étend en travers d'une section centrale importante de la région abdominale avant ou arrière dans un sens dans lequel la couche-culotte de type culotte doit être portée.

3. Panneau de préforme (1) pour une couche-culotte de type culotte qui est formé à partir d'un seul morceau de toile sensiblement en forme de W réalisé en coupant un côté d'un rectangle sous la forme de deux sommets et qui comporte une pré-section de tour de taille (4) destinée à tenir lieu de section de tour de taille, des pré-sections d'ouvertures pour les jambes (6) destinées à tenir lieu de sections d'ouvertures pour les jambes, et une pré-section entrejambe (5) destinée à tenir lieu de section entrejambe, le panneau de préforme comportant par ailleurs :
la pré-section de tour de taille (4) étant présente sous la forme d'une partie sensiblement rectangulaire, où un élément élastique (8) est mis en oeuvre dans la pré-section de tour de taille (4) d'une manière extensible ;
les pré-sections d'ouvertures pour les jambes (6) étant présentes sous la forme d'une paire de trous et en des positions qui sont symétriques autour de l'axe de symétrie du panneau de préforme pour une couche-culotte de type culotte ; et
la pré-section entrejambe (5) étant présente sous la forme de deux sommets s'étendant depuis un bord inférieur de la pré-section de tour de taille sensiblement rectangulaire.

4. Panneau de préforme (20) pour une couche-culotte de type culotte qui est formé à partir d'une seule toile, la toile adoptant une forme sensiblement semi-circulaire et ayant une section entaille (21) sensiblement au niveau du centre d'une corde d'un patron semi-circulaire, et qui comporte une pré-section de tour de taille (4) destinée à tenir lieu de section de tour de taille, des pré-sections d'ouvertures pour les jambes (6) destinées à tenir lieu de sections d'ouvertures pour les jambes, et une pré-section entrejambe (5) destinée à tenir lieu de section entrejambe, le panneau de préforme (20) comportant par ailleurs :
la pré-section de tour de taille (4) et la pré-section entrejambe (5) étant situées comme une corde du patron semi-circulaire, où un élément élastique (8) est mis en oeuvre dans la pré-section de tour de taille (4) dans un état allongé ; et
les pré-sections d'ouvertures pour les jambes (6) étant présentes sous la forme de trous et en des positions qui sont symétriques autour de l'axe de symétrie (Y-Y) du panneau de préforme pour une couche-culotte de type culotte.

5. Panneau de préforme (30) pour une couche-culotte de type culotte qui est formé à partir d'une seule toile, la toile adoptant une forme sensiblement rectangulaire et ayant une partie en saillie sensiblement trapézoïdale faisant saillie depuis un centre d'un bord inférieur du bord rectangulaire, et qui comporte une pré-section de tour de taille (31) destinée à tenir lieu de section de tour de taille, des pré-sections d'ouvertures pour les jambes destinées à tenir lieu de sections d'ouvertures pour les jambes, et une pré-section entrejambe (32) destinée à tenir lieu de section entrejambe, le panneau de préforme (30) comportant par ailleurs :
la pré-section de tour de taille (31) étant présente sous la forme d'une partie sensiblement rectangulaire, où un élément élastique (8) est mis en oeuvre dans la pré-section de tour de taille (31) d'une manière extensible ;
les pré-sections d'ouvertures pour les jambes étant présentes sous la forme d'une entaille en forme de sommet (33) dans la pré-section de tour de taille (31) et la pré-section entrejambe (32) et enjambant la partie en saillie sensiblement trapézoïdale ; et
la pré-section entrejambe (32) étant présente sous la forme d'une partie en saillie sensiblement trapézoïdale s'étendant depuis un centre d'un bord inférieur de la pré-section de tour de taille sensiblement rectangulaire (31).

6. Couche-culotte de type culotte selon la revendication 1 ou la revendication 2, dans laquelle l'absorbant (7) est divisé en deux depuis un centre de celui-ci jusqu'à une extrémité longitudinale de celui-ci dans un sens de la largeur ; une section non divisée (7a) de l'absorbant est située au niveau d'une section intermédiaire entre la paire d'ouvertures pour les jambes (14) dans la région abdominale avant (100), et les parties divisées (7b) de l'absorbant sont mises en oeuvre au niveau d'un centre important de la région abdominale arrière (101) et des deux côtés et le long de la section collée (10).

7. Couche-culotte de type culotte selon la revendication 1 ou la revendication 2, dans laquelle l'absorbant (7) est divisé en deux depuis un centre de celui-ci jusqu'à une extrémité longitudinale de celui-ci dans un sens de la largeur ; une section non divisée (7a) de l'absorbant est située au niveau d'un centre important de la région abdominale arrière (101), et les parties divisées (7b) de l'absorbant sont mises en oeuvre au niveau d'une section intermédiaire entre les ouvertures pour les jambes (14) dans la région abdominale avant (100) et des deux côtés et le long de la section collée (10).

8. Couche-culotte de type culotte selon la revendication 1 ou la revendication 2, dans laquelle l'absorbant (7) est mis en oeuvre de manière à s'étaler en travers des régions abdominales avant et arrière (100, 101) par le biais de la section entrejambe (12), pour de ce fait recouvrir la section collée (10).

9. Couche-culotte de type culotte selon l'une quelconque des revendications 6 à 8, dans laquelle une paroi antifuite (16) est mise en oeuvre le long des bords longitudinaux de l'absorbant (7) sur toute la longueur de celui-ci.

10. Panneau de préforme pour une couche-culotte de type culotte selon la revendication 3 ou la revendication 5, dans lequel l'élément élastique (8) est mis en oeuvre le long d'un bord supérieur de la pré-section de tour de taille (4) d'une manière étendue.

11. Panneau de préforme pour une couche-culotte de type culotte selon la revendication 4, dans lequel l'élément élastique (8) est mis en oeuvre le long d'un pourtour extérieur de la pré-section de tour de taille (4) d'une manière extensible.

12. Couche-culotte de type culotte selon la revendication 3 ou la revendication 4, dans laquelle l'élément élastique (8) est mis en oeuvre le long des bords périphériques des pré-sections d'ouvertures pour les jambes (6) d'une manière extensible.

13. Couche-culotte de type culotte selon la revendication 5, dans laquelle l'élément élastique (8) est mis en oeuvre le long d'un bord périphérique de l'entaille en forme de sommet (33) en travers de la pré-section de tour de taille (31) et de la pré-section entrejambe (32) dans un état allongé.
